# EUROPEAN PATENT APPLICATION

(11) **EP 1 875 877 A1**
(43) Date of publication of application: **09.01.2008**
(21) Application number: 06013891.4
(22) Date of filing: 05.07.2006
(51) Int. Cl.: A61B 19/00, A61N 1/372

(54) **Method for a foolproof identification of a technical device and such technical device**

(71) Applicant: BIOTRONIK CRM Patent AG, 6341 Baar (CH)
(72) Inventor: Bungartz, Jörn, 13086 Berlin (DE); Kraus, Alexander, Dr., 10407 Berlin (DE)
(74) Representative: Lindner-Vogt, Karin L.

(57) **Abstract**

A method for a foolproof identification of a technical device in a data system comprises the steps of
- providing each specimen of the technical device (2) with an identification value (SN) which is representative for said certain specimen of said technical device (2),
- automatically generating a defined checksum value (PID) which is determined from said identification value (SN) by means of a checksum algorithm,
- automatically querying the identification value (SN) and checksum value (PID) when the certain specimen of the technical device (2) is to be entered into the data system (3), and
- said data system (3) checking the queried identification value (SN) and the checksum value (PID) on the basis of the checksum algorithm as to the correctness of the entered identification value (SN).

## Description

The invention refers to a method for a foolproof identification of a technical device, in particular of a medical implant, in a data system and to such a technical device.

As a background of the invention reference is made to the field of medical implants, like heart pacemakers and modern monitoring systems for same which include so-called home monitoring service centers. The latter monitor a patient who wears a medical implant like a heart pacemaker by means of modern telecommunication techniques, like mobile phone functionalities. Each patient who wants to be incorporated into the home monitoring system has to be logged on to this data system. During this application session e.g. a physician is asked to enter an identification value which is representative for the certain specimen of the medical implant, e.g. the serial number of this implant. This serial number is read from a label or from the manual accompanying the medical implant and the number is entered into the home monitoring system by the physician. Entering the serial number into the data system can be faulty. Nevertheless the data system accepts the serial number as valid. This results in a mismatch between the real world situation and the database content of the data system - whereas the registered specimen of the medical implant in realiter bears e.g. the serial number 12345, the data system holds the faulty number 12346 entered by the physician after surgery.

To overcome this problem it is known that the home monitoring service center as data system not only queries the serial number of the device but also the device type. In this case the device serial number can be cross-checked in the Factory Output Database (FOD) against the type for correctness. This process, however, is time consuming and uncomfortable to the user. It is also insecure because the erroneous serial number often refers to an implant of the same type, as serial number ranges are reserved for a given implant type.

Going out from the problems discussed above it is an object of the invention to provide for a method for a foolproof identification of a technical device which enables a data system to detect errors in the external processing, e.g. reading, communicating, typing and the like by operating persons in connection with a device serial number.

This object is achieved by an identification method comprising the steps of the characterizing part of claim 1, i.e.
- providing each specimen of the technical device with an identification value which is representative for said certain specimen of said technical device,
- automatically generating a defined checksum value which is determined from said identification value by means of a checksum algorithm,
- automatically querying the identification value and checksum value when the certain specimen of the technical device is to be entered into the data system, and
- said data system checking the queried identification value and the checksum value on the basis of the checksum algorithm as to the correctness of the entered identification value.

As can readily be seen the data system utilizing the identification value of the technical device, e.g. the serial number, is provided with a control means which enables the system to detect when a faulty identification value is entered into the system. This means that compliance on the operator's part is not needed. Especially the most common mistakes when communicating or entering data into computer systems, like singular digit errors or swapping of two adjacent digits, are recognized. As it is highly improbable that such unintentional errors will result in a valid pair of an identification value and checksum value, such errors can effectively be prevented without looking up the serial number or checksum in a database for the technical devices implemented with the identification method according to the invention. It is only necessary that the checksum is generated by applying a suitable generation algorithm to the identification value. As long as the checksum algorithm is not changed, a given identification value will always produce the same checksum value. Thus any data system using this checksum algorithm can calculate a matching checksum value for an entered identification value. By means of comparing the calculated checksum value to the entered checksum value the data system can determine whether or not the entered values are correct.

Preferred embodiments of the identification method are given in the depending claims 2 through 8.

The invention further refers to a technical device, in particular a medical implant according to claim 9, which is identifiable based on aforesaid method in a foolproof manner. Accordingly, such technical device is provided with an identification value and a defined checksum value which is determined from said identification value by means of a checksum algorithm.

Further features, details and advantages of the identification method and technical device according to the invention will become apparent from the following description of preferred embodiments. In the drawings:
- Fig. 1: is a schematic flowchart representing an error detection in connection with a faulty entered serial number of a medical implant, and
- Fig. 2 - 4: are schematic diagrams reflecting different embodiments of the foolproof identification handling of a medical implant.

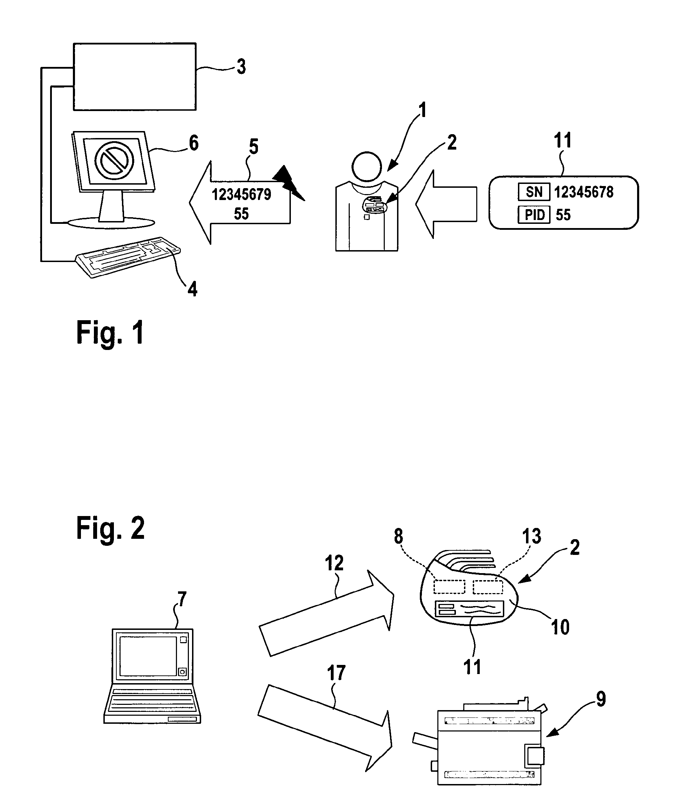

Referring to Fig. 1 a patient 1 receives a medical implant 2, e.g. a heart pacemaker which is provided with a serial number SN, namely "12345678", and a two-digit checksum value PID "55". As will be described below the checksum value PID is automatically generated by means of a defined checksum algorithm.

Modern medical implants 2 are able to be part of a remote control and monitoring system which is provided by a known data system 3 in a so-called home monitoring service center.

Now to introduce the patient 1 with the medical implant 2 to the home monitoring service center data system 3 it is commonly necessary to enter the serial number SN into the data system 3 via a keyboard 4. According to the invention not only the serial number SN, but also the checksum value PID with the value "55" is to be read from the type label (engraving 11) and entered into the data system 3, which is symbolized by the arrow 5. As is inscripted into this arrow 5 a wrong serial number "12345679" together with the checksum value PID of "55" was entered. Now the data system 3 uses the basic checksum algorithm to determine a checksum value based on the (faulty) serial number "12345679" which will be different from the correct checksum value PID of "55". The data system 3 thus determines a mismatch between the entered serial number SN and the calculated checksum value and thus generates a refusal message on the monitor 6. Thus the person having entered the wrong serial number is informed about this fact and can do a retry. When the correct serial number "12345678" together with the checksum value PID of "55" is entered, the data system 3 also defines the checksum value to be "55" and thus determines a match between the entered values. Inasmuch the serial number SN is accepted and stored in the data system 3 for further use within the home monitoring service center.

Referring to Fig. 2 the initialization of a certain specimen of the medical implant 2 is explained. During production of the medical implant 2 (see arrow 12) a production programmer 7 generates a unique serial number SN for the certain medical implant 2. This serial number SN is written to the memory 8 of the medical implant 2. Furtheron the production programmer 7 calculates the checksum value PID matching to the serial number SN and initializes a printer 9 (see arrow 17) to produce labels, manuals and/or shipping documents for the certain specimen of the medical implant 2 reflecting the serial number SN and the checksum value PID. Furtheron both these values SN and PID can be applied to the housing 10 of the medical implant 2 by means of an engraving 11 which is also represented in Fig. 1 to be read by the physician entering the values SN and PID into the data system 3.

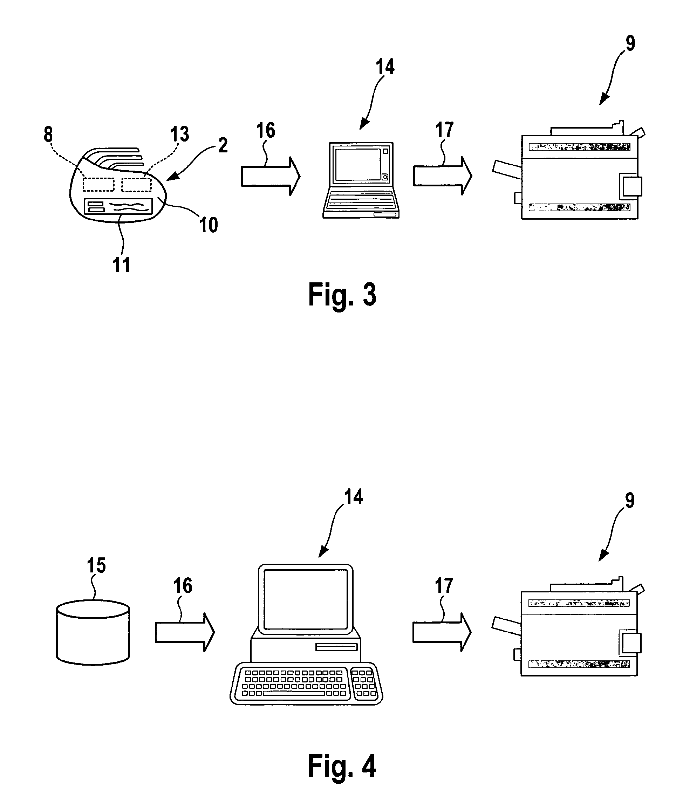

Referring to Fig. 3 a variant of the identification method is reflected. The medical implant 2 in this embodiment already holds a serial number SN in its memory which is connected with a transceiver means 13 commonly being part of the medical implant 2. Now a programmer 14 reads the stored serial number SN via short range telemetry, therefore the serial number entered into the programmer can be assumed to be correct. The programmer 14 then calculates the checksum value PID and again initiates the printer 9 (see arrow 17) to release labels, manuals and/or shipping documents for said specimen of the medical implant 2 reflecting its serial number and the checksum value. Thus all following handlings of the serial number SN by the data system 3 e.g. in connection with a home monitoring service center, are protected by the foolproof identification on the basis of the serial number SN and the checksum value PID.

Finally, as is depicted in Fig. 4, it is possible that a computer or programmer 14 is enabled to calculate said checksum value PID matching an implant serial number SN that is retrieved from a database 15 (or any other remote system). The programmer 14 reads (see arrow 16) the serial number SN and based on this calculates the checksum value and initiates the printer 9 (see arrow 17) again to print labels, manuals and/or shipping documents provided with the serial number SN and checksum value PID.

Due to the foregoing the checksum usage is to be summarized as follows:
- The checksum value PID can be used in remote monitoring systems where a medical implant has to be registered in the system using the serial number SN of the device 2. The user usually types in the serial number SN while reading it from a label, programmer printout or hand-written note. When forcing the user to enter the checksum as well, typing mistakes can be detected.
- Customer services are regularly confronted with inquiries as concerning information regarding an implantable device with a given serial number SN. During an inquiry the serial number is cited e.g. on the phone. When also a checksum value is to be transmitted, transmission errors e.g. due to misunderstanding or bad line quality are prevented.
- When doing surveys or clinical studies normally forms have to be filled requesting (amongst other things) the serial number SN of a medical implant 2. When also requesting the checksum value PID errors in the entered implant serial number SN can be detected and a correction of the data can then be requested.

Finally it is to be noted that the serial number SN and the checksum value PID can be combined to a unitary identification check value in which one continuous number inherently being divided in a serial number part and a checksum value part not showing up to the user. For example such combined unitary identification check value formed of the serial number SN and the checksum value PID of the medical implant 2 according to Fig. 1 to 3 would read "1234567855". In case such unitary identification check value is entered into the data system the latter splits up this value into the serial number SN of "12345678", calculates the checksum value from this number to be "55" and compares same to the last two digits "55" of the unitary identification check value with the result that the entered value is correct.

## Claims

1. Method for a foolproof identification of a technical device, in particular of a medical implant, in a data system **characterized by** the steps of
- providing each specimen of the technical device (2) with an identification value (SN) which is representative for said certain specimen of said technical device (2),
- automatically generating a defined checksum value (PID) which is determined from said identification value (SN) by means of a checksum algorithm,
- automatically querying the identification value (SN) and checksum value (PID) when the certain specimen of the technical device (2) is to be entered into the data system (3), and
- said data system (3) checking the queried identification value (SN) and the checksum value (PID) on the basis of the checksum algorithm as to the correctness of the entered identification value (SN).

2. Method according to claim 1, **characterized in that** the identification value (SN) and the checksum value (PID) are provided by a production programming device (7), whereby the identification value (SN) and / or the checksum value (PID) are stored into a device memory (8), are applied to the housing (10) of the device (2) and/or are printed onto shipping documents and/or device labels for each specimen of said technical device (2).

3. Method according to claim 1, **characterized in that** for providing for the checksum value (PID) the identification value (SN) stored in a transceiver means (13) of the device (2) is read-in by a programming device (14) via short range telemetry which programming device (14) calculates the checksum value (PID) from the identification value (SN), whereby both the identification value (SN) and the checksum value (PID) are stored into a device memory (8), are applied to the housing (10) of the device (2) and/or are printed onto shipping documents and device labels for each specimen of said technical device (2).

4. Method according to claim 1, **characterized in that** the checksum value (PID) is a two-digit-value.

5. Method according to claim 1, **characterized in that** the identification value (SN) and the checksum value (PID) are combined to a unitary identification check value.

6. Method according to claim 1, **characterized in that** the data system (3) generates a refusal message upon determining a mismatch between the entered identification value (SN) and the checksum value (PID).

7. Method according to claim 1, **characterized in that** the data system (3) accepts the identification value (SN) upon determining a match between the entered identification value (SN) and the checksum value (PID).

8. Method according to claim 1, **characterized in that** the data system is a remote monitoring system (3) for medical implants (2).

9. Technical device, in particular a medical implant, **characterized in that** based on the method according to one of the claims 1 to 8 each specimen of the device (2) is
- provided with an identification value (SN) and a defined checksum value (PID) which is determined from said identification value (SN) by means of a checksum algorithm and
- identifiable by a data system (3).

10. Technical device according to claim 9, **characterized in that** the technical device is a medical implant (2) and the data system (3) is remote monitoring system for medical implants (2).
